# EUROPEAN PATENT APPLICATION

(11) **EP 3 042 601 A1**
(43) Date of publication of application: **13.07.2016**
(21) Application number: 14865638.2
(22) Date of filing: 08.10.2014
(51) Int. Cl.: A61B 1/00, G02B 23/24

(54) **CURVE PART OF ENDOSCOPE**

(30) Priority: 29.11.2013 JP 2013248475
(71) Applicant: Olympus Corporation, Hachioji-shi Tokyo 192-8507 (JP)
(72) Inventor: OSAKI, Itaru, Tokyo 192-8507 (JP); KONDO, Yoshinori, Tokyo 192-8507 (JP)
(74) Representative: Gunzelmann, Rainer
(86) International application number: PCT/JP2014/076986
(87) International publication number: WO 2015/079809

(57) **Abstract**

A bending portion of an endoscope capable of realizing optimum bending forms corresponding to purposes by a simple configuration is provided. For this purpose, knot rings for upward/downward bending and knot rings for left/right bending arrayed in a first bending area A1 set on a distal end side of a bending portion 6 are configured with second knot rings 32 on which a pair of projecting portions 32b are arranged at axial targets relative to a central axis Oi of an insertion portion 2; knot rings for left/right bending arrayed on a second bending area A2 set on a proximal end side of the bending portion 5 are configured with second knot rings 32; and knot rings for upward/downward bending excreted into the second bending area are configured with third knot rings 33 on which a pair of projecting portions 33b are offset upward from axial target positions relative to the central position Oi of the insertion portion 2 by a predetermined amount of offset.

## Description

### Technical Field

The present invention relates to a bending portion of an endoscope in which a plurality of knot rings are arrayed along a central axis of an insertion portion.

### Background Art

In general, a bending portion provided in an insertion portion of an endoscope has a knot ring group (a bending tube) in which a plurality of knot rings (bending pieces) are arrayed in line along a central axis of the insertion portion, as its skeleton structure body. The respective knot rings constituting the bending tube are such that, for example, knot rings adjoining each other in front and behind are rotatably coupled with each other via a pivot pin. Further, a pulling wire routed from the operation portion of the endoscope and the like is coupled with a knot ring located at a distal end side (for example, a forefront) of the bending tube, and the bending tube can perform a bending motion in a predetermined direction by pulling force of the pulling wire.

By the way, in general, various kinds of internal components, such as various kinds of signal cables and light guide bundles, are inserted inside the bending tube configured as described above. The internal components have predetermined rigidity resistant to a bending motion. When the pulling force of the pulling wire is transmitted via the bending tube in the bending portion, a larger moment occurs on a proximal end side than on a distal end side in the internal components. Therefore, basically, the proximal end side of the bending portion in which the internal components are inserted bends preferentially.

On the other hand, various bending forms are required for the bending portion of the endoscope according to an applied luminal shape and the like. For example, in order to improve insertability of the endoscope into a lumen and the like with a small curvature radius, it is desirable to cause the distal end side of the bending portion to bend with a small curvature radius.

In comparison, for example, Japanese Patent Application Laid-Open Publication No. 2012-75659 (Patent Literature 1) discloses a technique for reducing the curvature radius on the distal end side of the bending portion by arraying knot rings on which a pair of coupling portions is formed at each of positions deviated from a radial direction on both ends in an axial direction, on the distal end side of the bending portion, and rotatably coupling coupling portions of adjoining knot rings with each other by a pin (a rivet).

However, in order to rotatably couple the coupling portions of adjoining knot rings in the technique disclosed in Japanese Patent Application Laid-Open Publication No. 2012-75659 (Patent Literature 1), it is necessary to arrange coupling portions constituting each pair at positions deviated from the radial direction in a state of being mutually parallel. Therefore, the structure is somewhat complicated, for example, the shape of the knot rings is a non-cylindrical shape.

The present invention has been made in view of the above situation, and an object is to provide a bending portion of an endoscope capable of realizing optimum bending forms corresponding to purposes by a simple configuration.

### Disclosure of Invention

### Means for Solving the Problem

A bending portion of an endoscope according to an aspect of the present invention is a bending portion of an endoscope including a knot ring group in which a plurality of knot rings are arrayed in line along a central axis of an insertion portion and a pulling wire capable of pulling the knot ring located on a distal end side of the knot ring group from a proximal end side, the knot ring group being caused to perform a bending motion by pulling force of the pulling wire, wherein each of the knot rings has a ring-shaped knot ring body and paired projecting portions projecting from one end face of the knot ring body and swingably contacting the other end face of another adjoining knot ring body; and as for at least one of the respective knot rings, an axial line connecting swing fulcrums formed at respective portions of contact with the other end face of the other knot ring body adjoining the pair of projecting portions is offset relative to a central axis of the insertion portion by an amount of offset different from an amount of offset of an axial line connecting the swing fulcrums of any other of the knot rings.

### Brief Description of the Drawings

Fig. 1 is a perspective view of an endoscope;
Fig. 2 is a horizontal-axis cross sectional view of a distal end side of an insertion portion;
Fig. 3 is a vertical-axis cross sectional view of the distal end side of the insertion portion;
Fig. 4 is an A-A cross sectional view of Fig. 3;
Fig. 5 is a B-B cross sectional view of Fig. 3;
Fig. 6 is a C-C cross sectional view of Fig. 3;
Fig. 7 is a D-D cross sectional view of Fig. 3;
Fig. 8 is a perspective view showing a distal end side of a light guide;
Fig. 9 is a perspective view showing an image pickup unit;
Fig. 10 is a top view of a bending tube;
Fig. 11 is a side view of the bending tube;
Fig. 12A is a side view of a second knot ring;
Fig. 12B is a perspective view of the second knot ring;
Fig. 13A is a side view of a third knot ring;
Fig. 13B is a perspective view of the third knot ring;
Fig. 14 is a top view showing bending states of a knot ring group in right and left directions in a state that a bending angle is small;
Fig. 15 is a top view showing bending states of the knot ring group in the right and left directions when the bending angle is maximum;
Fig. 16 is a side view showing bending states of the knot ring group in upward and downward directions in the state that the bending angle is small;
Fig. 17 is a side view showing bending states in the upward and downward directions when the bending angle is maximum;
Fig. 18 relates to a first modification and is a side view of a fifth knot ring;
Fig. 19 relates to a second modification and is a side view of the bending tube;
Fig. 20A relates to a third modification and is a side view of a sixth knot ring;
Fig. 20B is a perspective view of the sixth knot ring;
Fig. 21 relates to the third modification and is a side view of the bending tube;
Fig. 22A relates to a fourth modification and is a side view of a seventh knot ring;
Fig. 22B is a perspective view of the seventh knot ring;
Fig. 23A relates to the fourth modification and is a side view of an eighth knot ring;
Fig. 23B is a perspective view of the eighth knot ring;
Fig. 24 relates to the fourth modification and is a side view of the bending tube;
Fig. 25A relates to a fifth modification and is a side view of a ninth knot ring;
Fig. 25B is a perspective view of the ninth knot ring;
Fig. 26 relates to the fifth modification and is a side view of the bending tube;
Fig. 27 relates to a sixth modification and is a side view of the bending tube;
Fig. 28 relates to a seventh modification and is a perspective view of a first knot ring accommodating an image pickup unit;
Fig. 29 relates to the seventh modification and is an E-E cross sectional view of Fig. 28;
Fig. 30 relates to the seventh modification and is an F-F cross sectional view of Fig. 28;
Fig. 31 relates to an eighth modification and is a cross sectional view of main portions of a distal end rigid portion;
Fig. 32 relates to a ninth modification and is a cross sectional view of main portions of the distal end rigid portion; and
Fig. 33 relates to a tenth modification and is a cross sectional view of main portions of the distal end rigid portion.

### Best Mode for Carrying Out the Invention

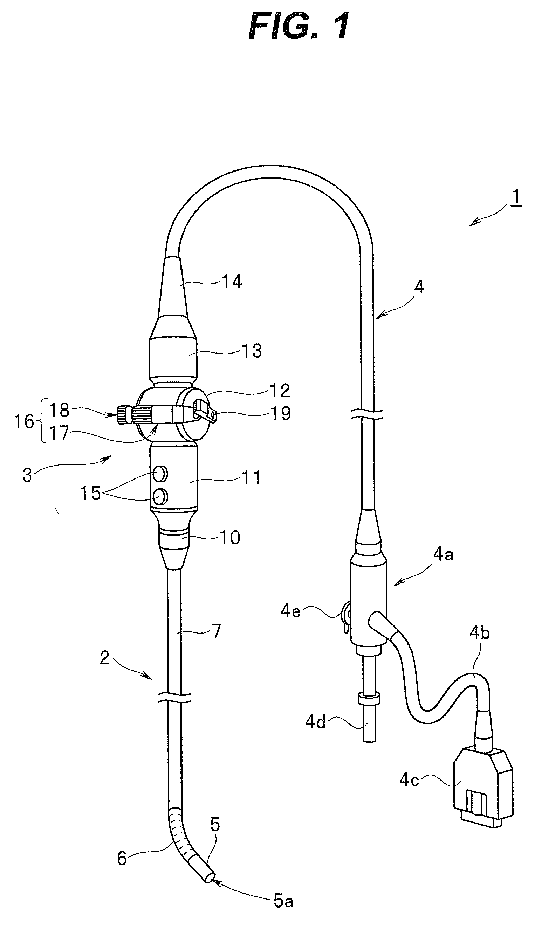

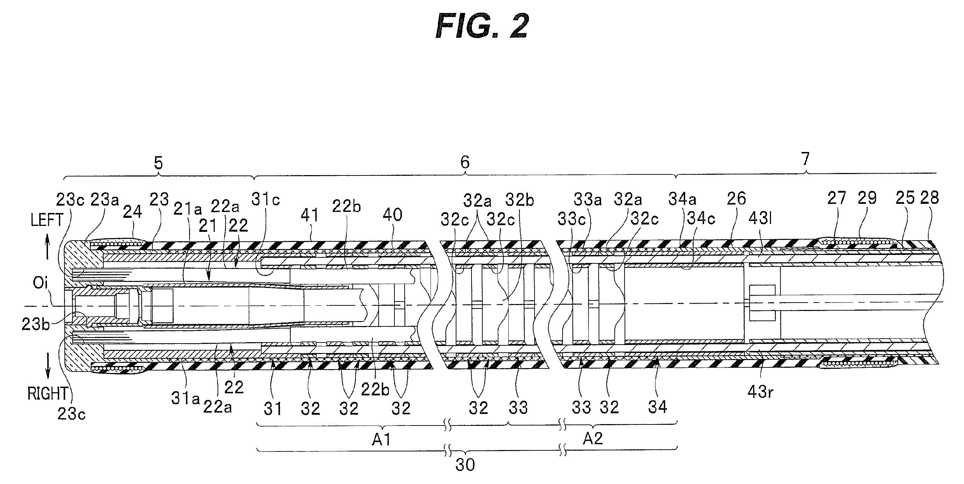

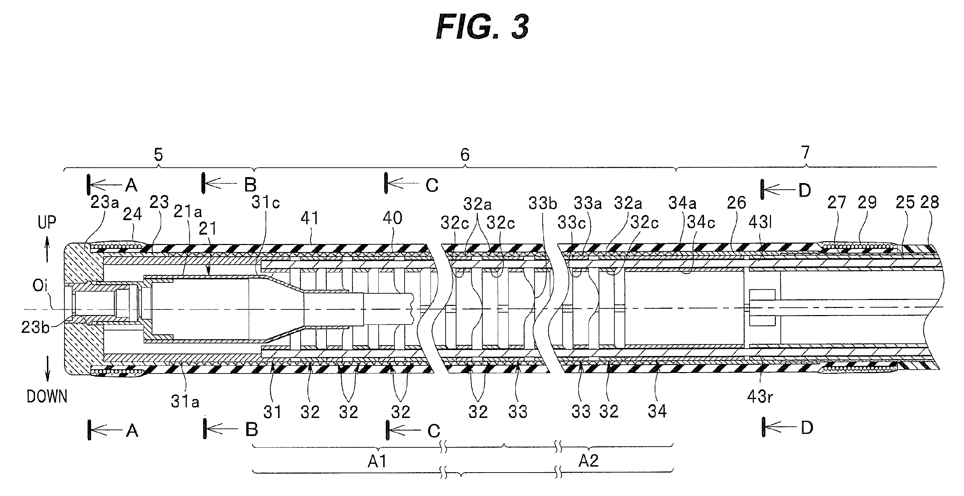

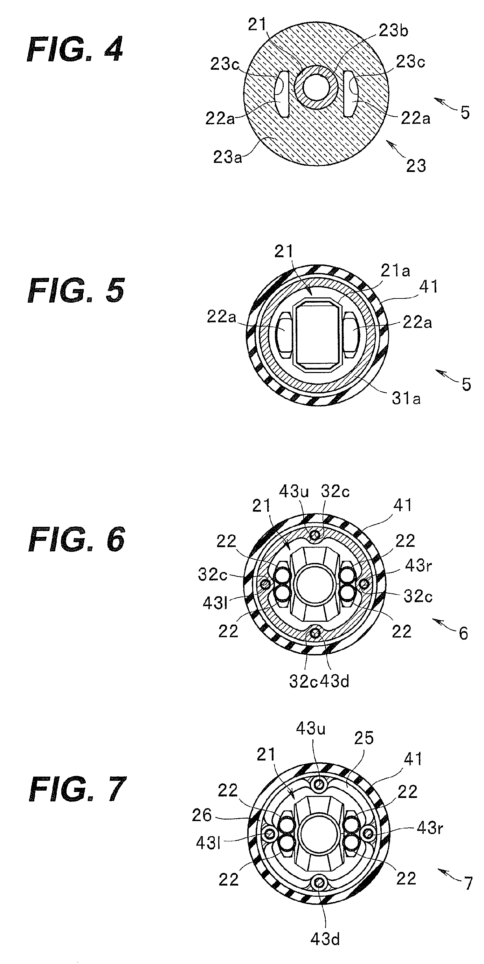

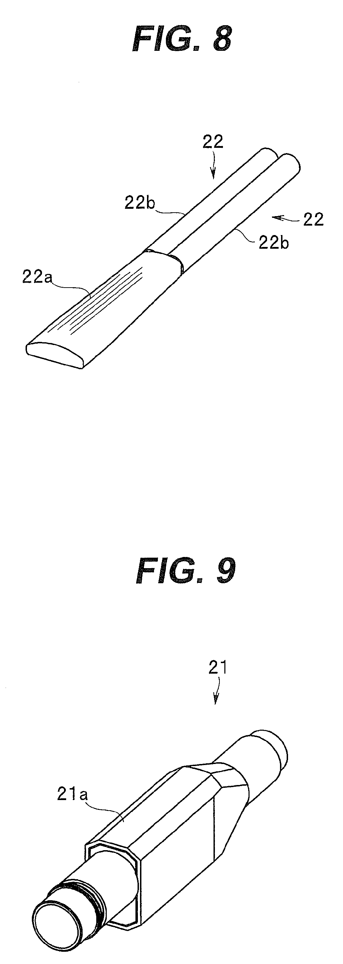

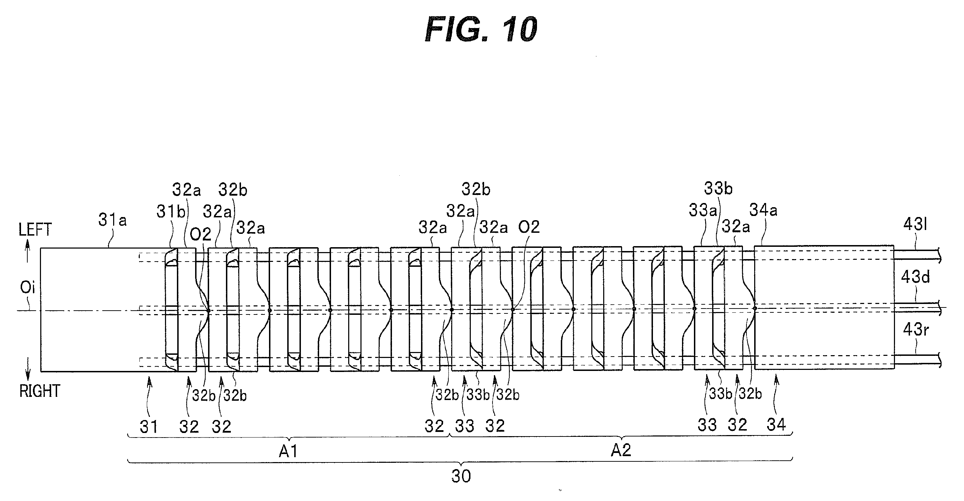

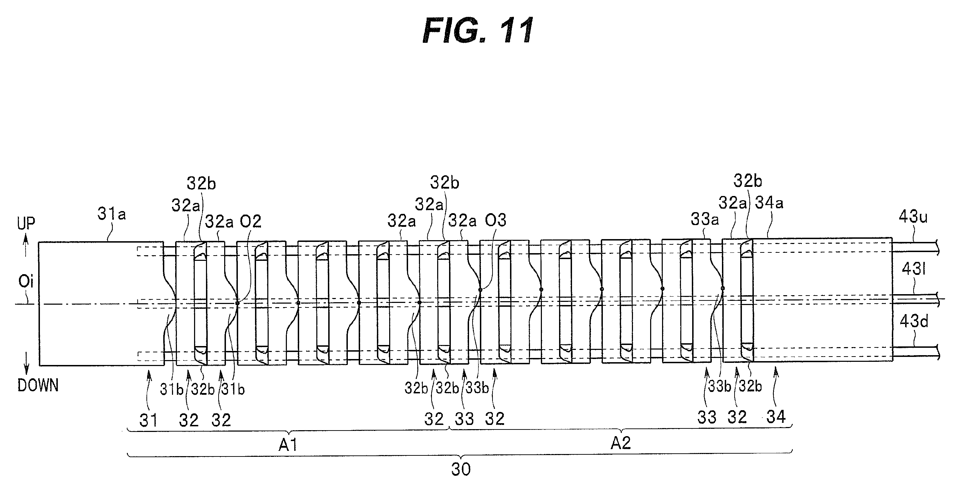

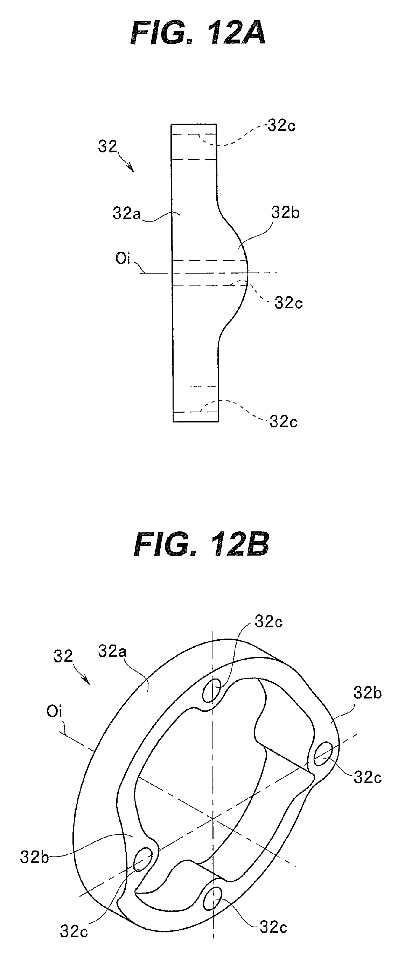

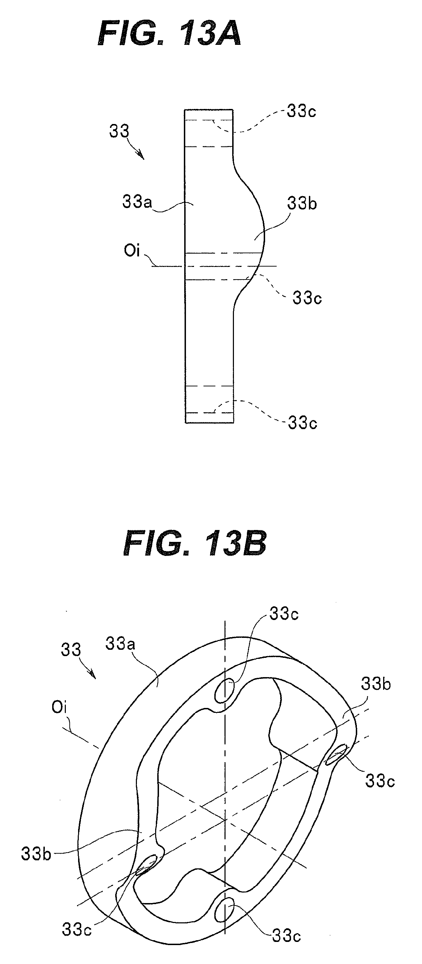

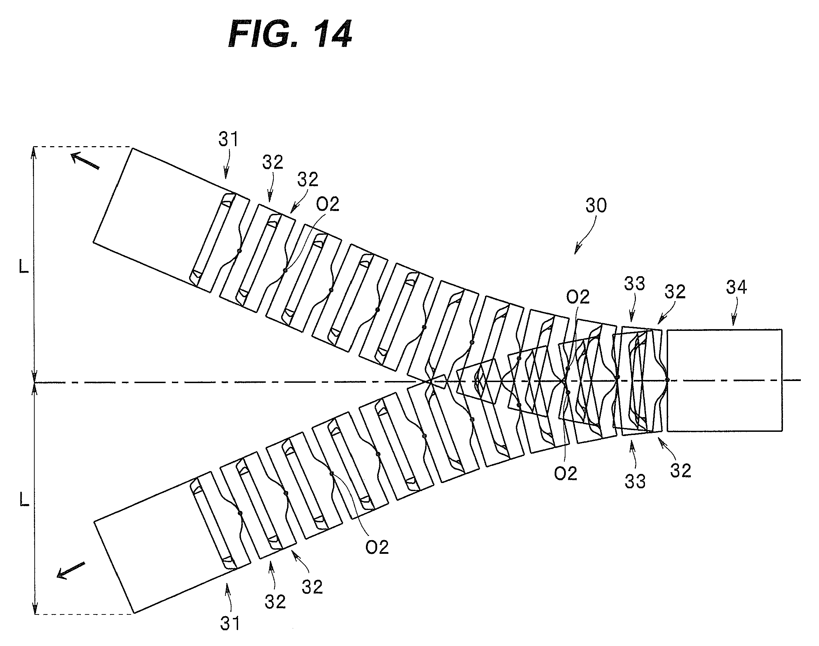

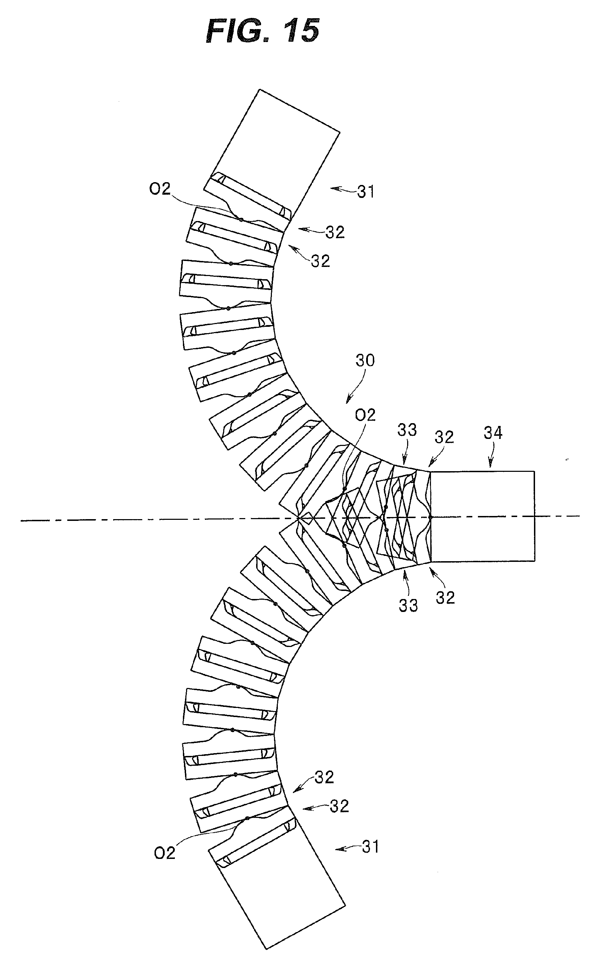

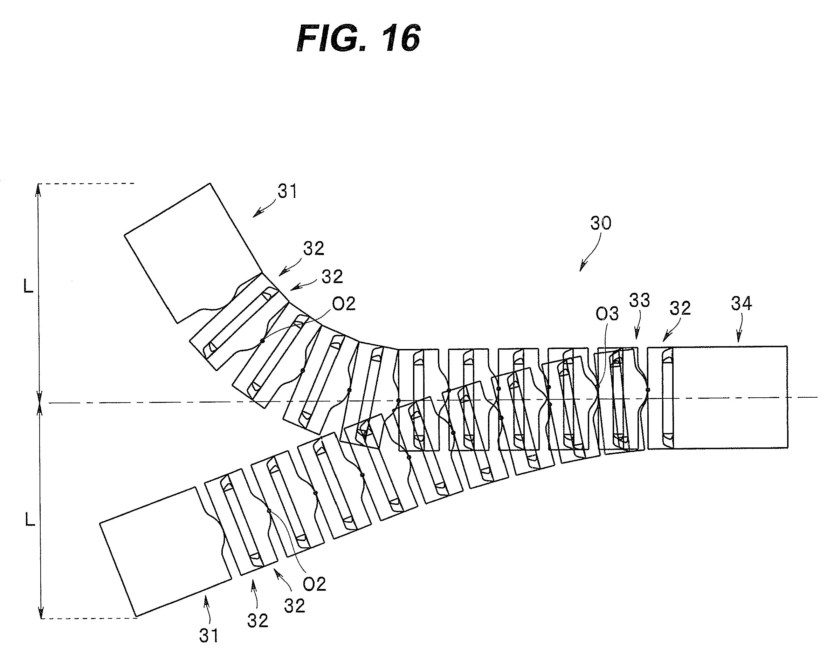

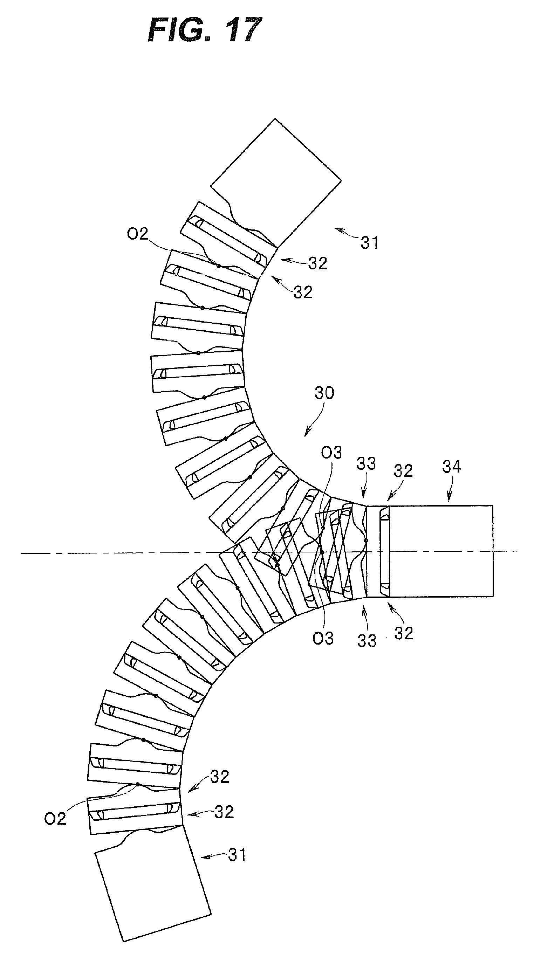

An embodiment of the present invention will be described with reference to drawings. The drawings relate to an embodiment of the present invention. Fig. 1 is a perspective view of an endoscope; Fig. 2 is a horizontal-axis cross sectional view of a distal end side of an insertion portion; Fig. 3 is a vertical-axis cross sectional view of the distal end side of the insertion portion; Fig. 4 is an A-A cross sectional view of Fig. 3; Fig. 5 is a B-B cross sectional view of Fig. 3; Fig. 6 is a C-C cross sectional view of Fig. 3; Fig. 7 is a D-D cross sectional view of Fig. 3; Fig. 8 is a perspective view showing a distal end side of a light guide; Fig. 9 is a perspective view showing an image pickup unit; Fig. 10 is a top view of a bending tube; Fig. 11 is a side view of the bending tube; Fig. 12A is a side view of a second knot ring; Fig. 12B is a perspective view of the second knot ring; Fig. 13A is a side view of a third knot ring; Fig. 13B is a perspective view of the third knot ring; Fig. 14 is a top view showing bending states of a knot ring group in right and left directions in a state that a bending angle is small; Fig. 15 is a top view showing bending states of the knot ring group in the right and left directions when the bending angle is maximum; Fig. 16 is a side view showing bending states of the knot ring group in upward and downward directions in the state that the bending angle is small; and Fig. 17 is a side view showing bending states of the knot ring group in the upward and downward directions when the bending angle is maximum.

As shown in Fig. 1, an endoscope 1 is configured being provided with an elongated insertion portion 2, an operation portion 3 and a universal cord 4. The operation portion 3 is connectedly provided on a proximal end side of the insertion portion 2. The operation portion 3 is connectedly provided at a proximal end of the insertion portion 2. The universal cord 4 is extended from a side portion on a proximal end side of the operation portion 3.

The insertion portion 2 is configured by a distal end rigid portion 5, a bending portion 6 and a flexible tube portion 7 being connectedly provided in that order from a distal end side.

In the present embodiment, an image pickup unit 21 provided with an image pickup device (a CCD, a CMOS or the like) is included in the distal end rigid portion 5, and a distal end portion of a light guide fiber bundle 22 is arranged on the distal end rigid portion 5 (see Figs. 2 and 3). An observation portion 5a is configured with them at a distal end face of the distal end rigid portion 5.

The bending portion 6 is configured having a knot ring group 30 (bending tube) in which a plurality of knot rings (a predetermined number of first knot rings 31, a predetermined number of second knot rings 32, a predetermined number of third knot rings 33 and a predetermined number of fourth knot rings 34) to be described later are arrayed in line (see Figs. 2 and 3). Respective adjoining knot rings of the knot ring group 30 are connectedly provided in a state of being capable of mutually swinging in a predetermined direction, and, thereby, the bending portion 6 is bendable, for example, in upward and downward directions and right and left directions. Note that, in description below, the upward and downward, and right and left directions of the endoscope 1 are directions conveniently defined, for example, with a state of an operator (a surgeon) grasping a predetermined portion of the operation portion 3 as a reference (alternatively, with upward and downward, and right and left directions of an image picked up by the image pickup unit 21 as a reference), and upward and downward, and right and left directions in a state of use do not necessarily correspond to actual upward and downward, and right and left directions.

The operation portion 3 is configured by a fixing ring portion 10, a sub-grip portion 11, an operation portion body 12, a main grip portion 13 and a connection member 14 being connectedly provided in that order from an insertion portion side.

The fixing ring portion 10 is a member connecting a distal end side of the operation portion 3 and the proximal end side of the insertion portion 2. The sub-grip portion 11 is arranged on a proximal end side of the fixing ring portion 10. On an outer surface of the sub-grip portion 11, operation members 15 such as a remote switch for controlling the image pickup device and the like of the observation portion 5a are provided on a top surface.

The operation portion body 12 is arranged on a proximal end side of the sub-grip portion 11. In an inside of the operation portion body 12, a bending operation mechanism portion, which is a mechanism portion for causing the bending portion 6 to perform a bending motion in the upward and downward, and right and left directions, and a bending portion braking operation portion, which is a mechanism portion for holding (locking) a bending state of the bending portion 6 (neither is shown). Further, outside the operation portion body 12, a bending operation lever 16 (an upward/downward bending operation lever 17 and a left/right bending operation lever 18) coupled with the bending operation mechanism portion is provided, and a bending lock lever 19 coupled with the bending braking mechanism portion is provided.

The main grip portion 13 is arranged on a proximal end side of the operation portion body 12. On a proximal end side of the main grip portion 13, the connection member 14 in a tapered shape having flexibility is connected. The connection member 14 is arranged so as to cover the universal cord 4 extended from the operation portion 3. Thereby, the connection member 14 prevents an end portion of the universal cord 4 from buckling near a portion of connection with the main grip portion 13.

Note that a plurality of endoscope components, such as the light guide fiber bundle 22, signal cables for transmitting various kinds of signals, shield cables and various kinds of tubes, are inserted in the insertion portion 2, operation portion 3 and universal cord 4 of the endoscope 1.

At an extension end of the universal cord 4, a light guide connector 4a capable of connecting the light guide fiber bundle 22 to a light source apparatus, which is an external apparatus not shown is provided. The light guide connector 4a is provided with a light guide tube 4d and a ventilation pipe sleeve 4e. A camera cable 4b branches from a side face of the light guide connector 4a. A connector 4c is provided at an extension end of the camera cable 4b, and the connector 4c is electrically connected to a control apparatus, which is an external apparatus not shown, a camera control unit provided with a signal processing circuit and the like.

Next, a detailed configuration of the insertion portion 2 with the bending portion 6 as a center will be described with reference to Figs. 2 to 17.

As shown in Figs. 2, 3, 10 and 11, the knot ring group 30 constituting the bending portion 6 of the present embodiment is configured having a first knot ring 31 arranged at a distal end, a fourth knot ring 34 arranged at a proximal end, and a plurality of second and third knot rings 32, 33 connectedly arranged between the first and fourth knot rings 31, 34. Further, a mesh tube 40 is sheathed over an outer circumference of the knot ring group 30, and the connectedly arranged state between adjoining knot rings among the respective knot rings 31 to 34 is kept by the mesh tube 40. Furthermore, a flexible bending rubber 41 is sheathed over an outer circumference of the mesh tube 40.

Here, the second and third knot rings 32 and 33 are configured in mutually different forms. By the second and third knot rings 32 and 33 being connectedly arranged in a predetermined array state, a first bending area A1 is formed on a distal end side of the bending portion 6, and a second bending area A2 which is different from the first bending area A1 in a bending form is formed on a proximal end side of the bending portion 6.

As shown in Figs. 10 and 11, the first knot ring 31 is configured with a metal member, for example, obtained by integrally forming a cylindrically shaped knot ring body 31a and paired projecting portions 31b projecting from a proximal end side of the knot ring body 31 a.

On an inner circumference on the proximal end side of the knot ring body 31 a, a wire stopping portion 31c is provided, for example, at each rotation position of 45° (see Figs. 2 and 3). That is, on the inner circumference of the knot ring body 31a, two pairs of wire stopping portions 31 c, which are axially symmetric to each other relative to a central axis of the knot ring body 31 a, are provided at each rotation position of 90°, and wire stopping portions 31c constituting each of the pairs are arranged along an upward/downward direction and left/right direction of the insertion portion 2.

Distal end portions of upward/downward pulling wires 43u and 43d which are alternately pulled or slackened by the bending operation mechanism portion in conjunction with an operation of the upward/downward bending operation lever 17 are fixed to two wire stopping portions 31 c facing each other in the upward/downward direction of the insertion portion 2, among the wire stopping portions 31c constituting the respective pairs. On the other hand, distal end portions of left/right pulling wires 431 and 43r which are alternately pulled or slackened by the bending operation mechanism portion in conjunction with an operation of the left/right bending operation lever 18 are fixed to two wire stopping portions 31 c facing each other in the right and left directions of the insertion portion 2.

Further, the pair of projecting portions 31b constituting the first knot ring 31 are configured, for example, with tongue-piece-shaped members having arc-shaped or parabolic-shaped projecting curved faces at their tip portions and projecting to a proximal end side as shown in Figs. 10 and 11. These projecting portions 31b are provided facing each other, for example, so as to be axially symmetric to each other relative to a central axis Oi of the insertion portion 2 (that is the central axis of the knot ring body 31a). Furthermore, the respective projecting portions 31b are provided at rotation positions corresponding to the wire stopping portions 31c of the left/right pulling wires 431 and 43r on a circumference of the knot ring body 31a (that is, the respective projecting portions 31b are arranged side by side in the right and left directions of the insertion portion 2).

Here, the knot ring body 31 a constituting the first knot ring 31 also has a function as an exterior tube which integrally accommodates distal end sides of the image pickup unit 21 and the light guide fiber bundle 22 by being extended to a distal end rigid portion 5 side and coupled with a distal end portion body 23.

When described more specifically, the distal end portion body 23 of the present embodiment is configured, for example, with a transparent resin member in a substantially cylindrical shape having a thick distal end wall portion 23a on its distal end side. The knot ring body 31a is coupled with an inner circumferential face of the distal end portion body 23 by being engaged with the inner circumferential face from the proximal end side.

Further, on the distal end wall portion 23a of the distal end portion body 23, a unit holding hole 23b configured with a through hole and a pair of light guide holding holes 23c configured with holes having a predetermined depth are provided (see Figs. 2 and 4). The distal end side of the image pickup unit 21 is inserted and held in the unit holding hole 23b, and wires 22a exposed from the distal end side of the light guide fiber bundle 22 are inserted and held in each of the light guide holding holes 23c.

On the other hand, an outer circumferential portion of the distal end portion body 23 is covered with the bending rubber 41 extending from a bending portion 6 side, and a distal end portion of the bending rubber 41 is bonded and fixed by a first bobbin portion 24.

Here, the unit holding hole 23b and the light guide holding holes 23c are provided at positions near to one another in order to realize reduction in the diameter of the insertion portion 2 and the like. Accordingly, the image pickup unit 21 and the light guide fiber bundle 22 of the present invention are integrally bonded and fixed in the knot ring body 31 a. In this case, in order to protect the internal components from interference of the light guide fiber bundle 22 and the like accommodated near to one another in the knot ring body 31 a, the image pickup unit 21 is covered with a reinforcing frame 21a made of metal (see Figs. 5 and 9). Further, though the light guide fiber bundle 22 is configured by the plurality of wires 22a being covered with an exterior tube 22b such as a thermal contraction tube (see Figs. 5 to 8), bonding, shaping or the like is not performed for the exterior tube 22b and the wires 22a in an area covered with the exterior tube 22b, on the distal end side of the light guide fiber bundle 22, and only the wires 22a in an area exposed from the exterior tube 22b are fitted in the light guide holding holes 23c in a state of being bondedly shaped, in order to efficiently arrange the light guide fiber bundle 22 in the knot ring body 31 a according to a shape and the like of the image pickup unit 21.

Note that the distal end portion body 23 has a function as a lens portion for emitting illuminating light transmitted by the light guide fiber bundle 22 to an outside, by being configured with transparent resin material or the like. Further, in order to improve chemical resistance and the like by covering the distal end portion body 23 made of transparent resin material with the bending rubber 41 as much as possible, the first bobbin portion 24 is set near to a distal end of the distal end portion body 23.

The fourth knot ring 34 is configured having, for example, a cylindrically shaped knot ring body 34a made of metal. On an inner circumference of the knot ring body 34a, a wire inserting portion 34c is provided, for example, at each rotation position of 45°. That is, on the inner circumference of the knot ring body 34a, two pairs of wire inserting portions 34c, which are axially symmetric to each other relative to a central axis of the knot ring body 34a, are provided at each rotation position of 90°, and wire inserting portions 34c constituting each of the pairs are arranged along an upward/downward direction and left/right direction of the insertion portion 2.

The upward/downward pulling wires 43u and 43d are inserted in two wire inserting portions 34c facing each other in the upward/downward direction of the insertion portion 2 among the wire inserting portions 34c constituting the respective pairs. On the other hand, the left/right pulling wires 431 and 43r are inserted in two wire inserting portions 34c facing each other in the left/right direction of the insertion portion 2.

Here, on a proximal end side of the knot ring body 34a constituting the fourth knot ring 34, a spiral tube 25 which constitutes the flexible tube portion 7 is connectedly arranged. The proximal end side of the knot ring body 34a and a distal end side of the spiral tube 25 are sheathed with a cylindrically shaped connection tube 26, and, thereby, the knot ring body 34a is coupled with the spiral tube 25. Further, an outer circumference of the spiral tube 25 is sheathed with a mesh tube 27, and, further, an outer circumference of the mesh tube 27 is sheathed, for example, with an outer cover 28 made of flexible resin. Further, for example, the bending rubber 41 extending from the bending portion 6 side is connectedly arranged at a distal end of the outer cover 28 on a flexible tube portion 7 side, and a proximal end portion of the bending rubber 41 is bonded and fixed by a second bobbin portion 29.

As shown in Figs. 12A and 12B, the second knot ring 32 is configured with a metal member obtained by integrally forming a ring-shaped knot ring body 32a and paired projecting portions 32b projecting from a proximal end side of the knot ring body 32a.

On an inner circumference of the knot ring body 32a, a wire inserting portion 32c is provided, for example, at each rotation position of 45°. That is, on the inner circumference of the knot ring body 32a, two pairs of wire inserting portions 32c, which are axially symmetric to each other relative to a central axis of the knot ring body 32a, are provided at each rotation position of 90°.

The pair of projecting portions 32b constituting the second knot ring 32 are configured, for example, with tongue-piece-shaped members having arc-shaped or parabolic-shaped projecting curved faces at their tip portions and projecting to a proximal end side as shown in Figs. 10 to 12B. These projecting portions 32b are provided facing each other, for example, so as to be axially symmetric to each other relative to the central axis Oi of the insertion portion 2 (that is the central axis of the knot ring body 32a). Furthermore, the respective projecting portions 32b are provided at rotation positions corresponding to respective wire inserting portions 32c of one of the two pairs of wire inserting portions 32c arranged at the axially symmetric positions, on the circumference of the knot ring body 32a (see Figs. 12A and 12B).

As shown in Figs. 13A and 13B, the third knot ring 33 is configured with a metal member obtained by integrally forming a ring-shaped knot ring body 33a and paired projecting portions 33b projecting from a proximal end side of the knot ring body 33a.

On an inner circumference of the knot ring body 33a, a wire inserting portion 33c is provided, for example, at each rotation position of 45°. That is, on the inner circumference of the knot ring body 33a, two pairs of wire inserting portions 33c, which are axially symmetric to each other relative to a central axis of the knot ring body 33a, are provided at each rotation position of 90°.

The pair of projecting portions 33b constituting the third knot ring 33 are configured, for example, with tongue-piece-shaped members having arc-shaped or parabolic-shaped projecting curved face at their tip portions and projecting to a proximal end side as shown in Figs. 10, 11, 13A and 13B. These projecting portions 33b are provided facing each other, for example, at positions offset from positions which are axially symmetric to each other relative to the central axis Oi of the insertion portion 2 (that is, the central axis of the knot ring body 33a) by a predetermined amount. Furthermore, each projecting portion 33b is provided at such a position that an axial line connecting tips of the respective projecting portions 33b and an axial line connecting respective wire inserting portions 33c of one of the two pairs of wire inserting portions 33c arranged at the axially symmetric positions are in parallel to each other (see Fig. 13B).

Among the second and third knot rings 32 and 33, for example, only second knot rings 32 are arrayed in the first bending area A1 on the knot ring group 30, as shown in Figs. 10 and 11. That is, in the first bending area A1, for example, second knot rings 32 on which projecting portions 32b are arranged in the left/right direction of the insertion portion 2 and second knot rings 32 on which projecting portions 32b are arranged in the upward/downward direction of the insertion portion 2 are arrayed alternately. Furthermore, in respective wire inserting portions 32c of the respective second knot rings 32, corresponding upward/downward pulling wires 43u and 43d or left/right pulling wires 431 and 43r are inserted.

In the first bending area A1, the second knot rings 32 on which the projecting portions 32b are arranged in the left/right direction of the insertion portion 2 function as knot rings for bending in the upward and downward directions. By a pulling or slackening operation of the upward/downward pulling wires 43u and 43d, each of the second knot rings 32 can swing in the upward and downward directions with a contact portion between each projecting portion 32b of the second knot ring 32 and a distal end face of the knot ring body 32a of another adjoining second knot ring 32 as a swing fulcrum. On the other hand, in the first bending area A1, the second knot rings 32 on which the projecting portions 32b are arranged in the upward/downward direction of the insertion portion 2 function as knot rings for bending in the right and left directions. By a pulling or slackening operation of the left/right pulling wires 431 and 43r, each of the second knot rings 32 can swing in the right and left directions with a contact portion between each projecting portion 32b of the second knot ring 32 and a distal end face of the knot ring body 32a of another adjoining second knot ring as a swing fulcrum.

In this case, since the pair of projecting portions 32b constituting the second knot ring 32 are provided at the positions axially symmetric to each other relative to the central axis Oi of the insertion portion 2, an axial line 02 connecting contact portions to be swing fulcrums, in the respective knot rings for upward/downward bending and for left/right bending arrayed in the first bending area A1, exists at a position where an amount of offset relative to the central axis Oi of the insertion portion 2 is substantially 0 (see Figs. 10 and 11).

Further, in the second bending area A2, for example, second knot rings 32 and third knot rings 33 are arrayed alternately. That is, in the second bending area A2, for example, third knot rings 33 on which projecting portions 33b are arranged at positions in the left/right direction of the insertion portion 2 which are offset upward, and second knot rings 32 on which projecting portions 32b are arranged in the upward/downward direction of the insertion portion 2 are arranged alternately. Furthermore, in respective wire inserting portions 32c and 33c of the respective second and third knot rings 32 and 33, the corresponding upward/downward pulling wires 43u and 43d or left/right pulling wires 431 and 43r are inserted.

In the second bending area A2, the third knot rings 33 on which the projecting portions 33b are arranged in the left/right direction of the insertion portion 2 function as knot rings for bending in the upward and downward directions. By a pulling or slackening operation of the upward/downward pulling wires 43u and 43d, each of the third knot rings 33 can swing in the upward and downward directions with a contact portion between each projecting portion 33b of the third knot ring 33 and a distal end face of the knot ring body 32a of another adjoining second knot ring 32 as a swing fulcrum. On the other hand, in the second bending area A2, the second knot rings 32 on which the projecting portions 32b are arranged in the upward/downward direction of the insertion portion 2 function as knot rings for bending in the right and left directions. By a pulling or slackening operation of the left/right pulling wires 431 and 43r, each of the second knot rings 32 can swing in the right and left directions with a contact portion between each projecting portion 32b of the second knot ring 32 and a distal end face of the knot ring body 33a of another adjoining third knot ring 33 as a fulcrum.

In this case, since the pair of projecting portions 33b constituting the third knot ring 33 are offset from the central axis Oi of the insertion portion 2, an axial line 03 connecting contact portions to be swing fulcrums, in the respective knot rings for upward/downward bending arrayed in the second bending area, exists at a position offset upward from the central axis Oi of the insertion portion 2 by a predetermined amount of offset (see Fig. 11). On the other hand, since the pair of projecting portions 32b constituting the second knot ring 32 are provided at the positions axially symmetric to each other relative to the central axis Oi of the insertion portion 2, the axial line 02 connecting contact portions to be swing fulcrums, in the respective knot rings for left/right bending arranged in the second bending area A2, exists at a position where an amount of offset relative to the central axis Oi of the insertion portion 2 is substantially 0.

In the bending portion 6 of the insertion portion 2 configured as described above, for example, a pulling or slackening operation of the left/right pulling wires 431 and 43r is performed in conjunction with an operation of the left/right bending operation lever 18, the bending portion 6 is bent in the left/right direction.

In this case, when an amount of the left/right-direction operation of the left/right bending operation lever 18 is small, the proximal end side of the bending portion 6 is preferentially bent, for example, as shown in Fig. 14. That is, though second knot rings 32 are arrayed as knot rings for bending in the right and left directions in both of the first and second bending areas A1 and A2, mainly the proximal end side (the second bending area A2 side) of the bending portion 6 is preferentially bent due to influence of the internal components if the amount of the left/right-direction operation is small. Therefore, a curvature radius r effective at the time of insertion into a bent conduit becomes relatively large. Note that the effective curvature radius r of the bending portion 6 at a time when the distal end rigid portion 5 is moved in the left/right direction by a predetermined amount of movement L is equal on right and left and, for example, r = α.

On the other hand, when the amount of the left/right direction operation of the left/right bending operation lever 18 is large, the bending portion 6 is bent substantially uniformly as a whole, for example, as shown in Fig. 15. That is, though the bending portion 6 is bent from the proximal end side preferentially when being bent in the left/right direction as described above, the respective knot rings for left/right bending come to a limit of swing sequentially from the proximal end side if the amount of operation is large, and the bending portion 6 is bent substantially uniformly as a whole in the end. Note that the effective curvature radius r at a time when the bending portion 6 is bent in the left/right direction maximum is equal on right and left and, for example, r = β.

Further, for example, when a pulling or slackening operation of the upward/downward pulling wires 43u and 43d is performed in conjunction with an operation of the upward/downward bending operation lever 17, the bending portion 6 is bent in the upward/downward direction.

In this case, when an amount of an upward-direction operation is small, the distal end side of the bending portion 6 is preferentially bent, for example, as shown in Fig. 16. That is, the second knot rings 32 are arrayed in the first bending area A1 as knot rings for bending in the upward and downward directions, and the third knot rings 33 are arrayed in the second bending area A2 as knot rings for bending in the upward and downward directions. Since the axial line 03 to be a swing fulcrum is offset upward for the third knot rings 33, a distance from the swing fulcrum to an operation point at a time of bending upward is shorter in comparison with the second knot rings 32 for which the amount of offset of the axial line 02 to be a swing fulcrum is substantially zero. Therefore, the second knot rings 32 arrayed in the first bending area A1 as knot rings for upward/downward bending can swing upward with a smaller force than the third knot rings 33 arrayed in the second bending area A2 as knot rings for upward/downward bending, and the distal end side of the bending portion 6 is preferentially bent. Note that the effective curvature radius r of the bending portion 6 at a time when the distal end rigid portion 5 is moved upward by the predetermined amount of movement L is smaller than α and, for example, r = γ.

On the contrary, when an amount of a downward-direction operation is small, the proximal end side of the bending portion is preferentially bent, for example, as shown in Fig. 16. That is, since the axial line 03 to be a swing fulcrum is offset upward for the third knot rings 33, a distance from the swing fulcrum to an operation point at a time of bending downward is longer in comparison with the second knot rings 32 for which the amount of offset of the axial line 02 to be a swing fulcrum is substantially zero. Therefore, the third knot rings 33 arrayed in the second bending area A2 as knot rings for upward/downward bending can swing downward with a smaller force than the second knot rings 32 arrayed in the first bending area A1 as knot rings for upward/downward bending, and the proximal end side of the bending portion 6 is preferentially bent. Note that the effective curvature radius r of the bending portion 6 at a time when the distal end rigid portion 5 is moved downward by the predetermined amount of movement L is larger than α and, for example, r = δ.

On the other hand, when the amount of the upward/downward direction operation of the upper/lower bending operation lever 17 is large, the bending portion 6 is bent as a whole, for example, as shown in Fig. 17. That is, though the bending portion 6 is bent preferentially from the distal end side when being bent in the upward direction as described above, the respective knot rings for upward/downward bending come to a limit of swing sequentially from the distal end side if the amount of operation is large, and the bending portion 6 is bent as a whole in the end. Note that, as for the limit of swing of the respective knot rings for upward/downward bending, the limit is largest at a time of upward-direction swing of the third knot rings 33, second largest at a time of upward/downward-direction swing of the second knot rings, and third largest at a time of downward-direction swing of the third knot rings 33 because the swing fulcrums are offset. Therefore, the effective curvature radius r at a time when the bending portion 6 is bent in the upward direction maximum is larger than β and, for example, r = ε. Further, the effective curvature radius r at a time when the bending portion 6 is bent in the downward direction maximum is smaller than β, for example, r = ζ.

According to such an embodiment, by configuring knot rings for upward/downward bending and knot rings for left/right bending arrayed in the first bending area A1 set on the distal end side of the bending portion 6 with the second knot rings 32 on which the pair of projecting portions 32b are axially symmetrically arranged relative to the central axis Oi of the insertion portion 2, configuring knot rings for left/right bending arrayed in the second bending area A2 set on the proximal end side of the bending portion 6 with the second knot rings 32, and configuring knot rings for upward/downward bending arrayed in the second bending area with the third knot rings 33 on which the pair of projecting portions 33b are offset upward from axially symmetric positions relative to the central position Oi of the insertion portion 2 by the predetermined amount of offset, it is possible to realize optimum bending forms according to purposes by a simple configuration.

That is, for example, by configuring knot rings for upward/downward bending arrayed in the second bending area A2 set on the proximal end side of the bending portion 6 with the third knot rings 33, and offsetting the axial line 03 connecting swing fulcrums of the knot rings for upward/downward bending upward relative to the central axis Oi of the insertion portion 2, it is possible to cause the distal end side to be preferentially bent at the time of bending of the bending portion 6 in the upward direction. Thereby, it is possible to, at the time of bending in the upward direction, reduce the effective curvature radius r of the bending portion 6 even at beginning of bending or the like and improve the insertability into a lumen and the like with a small curvature radius.

In this case, the respective knot rings 31 to 34 are configured to be arranged being connected to an adjoining knot ring by contact of the projecting portions 31b to 33b in a manner that swing is possible, it does not happen that swing of the knot rings is especially prevented, for example, even if the projecting portions are offset relative to the central axis Oi of the insertion portion 2, and it is possible to realize optimum bending forms according to purposes by the simple configuration.

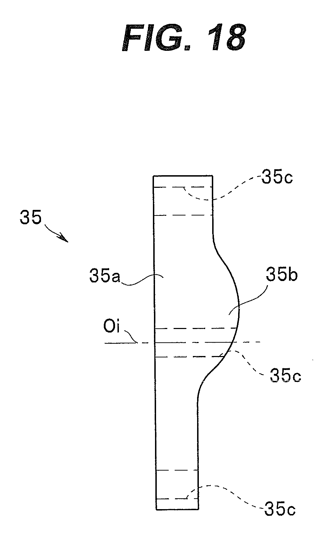

Next, a first modification of the present embodiment will be described with reference to Fig. 18. Note that, in the present modification, a fifth knot ring 35 is adopted instead of the third knot ring 33, for example, as shown in Fig. 18.

As shown in Fig. 18, the fifth knot ring 35 is configured with a metal member obtained by integrally forming a ring-shaped knot ring body 35a and paired projecting portions 35b projecting from a proximal end side of the knot ring body 35a.

On an inner circumference of the knot ring body 35a, a wire inserting portion 35c is provided, for example, at each rotation position of 45°. That is, on the inner circumference of the knot ring body 35a, two pairs of wire inserting portions 35c, which are axially symmetric to each other relative to a central axis of the knot ring body 35a, are provided at each rotation position of 90°.

The pair of projecting portions 35b constituting the fifth knot ring 35 are configured, for example, with tongue-piece-shaped members having arch-shaped or parabolic-shaped projecting curved face at their tip portions and projecting to a proximal end side as shown in Fig. 18. These projecting portions 35b are provided facing each other, for example, at positions offset from positions which are axially symmetric to each other relative to the central axis Oi of the insertion portion 2 (that is, the central axis of the knot ring body 35a) by a predetermined amount. Furthermore, each projecting portion 35b is provided at such a position that an axial line connecting tips of the respective projecting portions 35b and an axial line connecting respective wire inserting portions 35c of one of the two pairs of wire inserting portions 35c arranged at the axially symmetric positions are in parallel to each other.

Further, a height of a proximal end face of the knot ring body 35a (a width from a distal end face of the knot ring body 35a to the proximal end face) is set unequal on both sides of the projecting portions 35b. For example, as shown in Fig. 18, a side where the pair of projecting portions 35b are offset is set to be relatively higher than a side where the pair of projecting portions 35b are not offset.

By adopting such a configuration, it is possible to, for example, make an adjustment so that a limit of swing at a time of upward-direction swing of the fifth knot ring 35 and a limit of swing at a time of downward-direction swing become equal even when the projecting portions 35b are caused to be offset relative to the central axis Oi of the insertion portion 2.

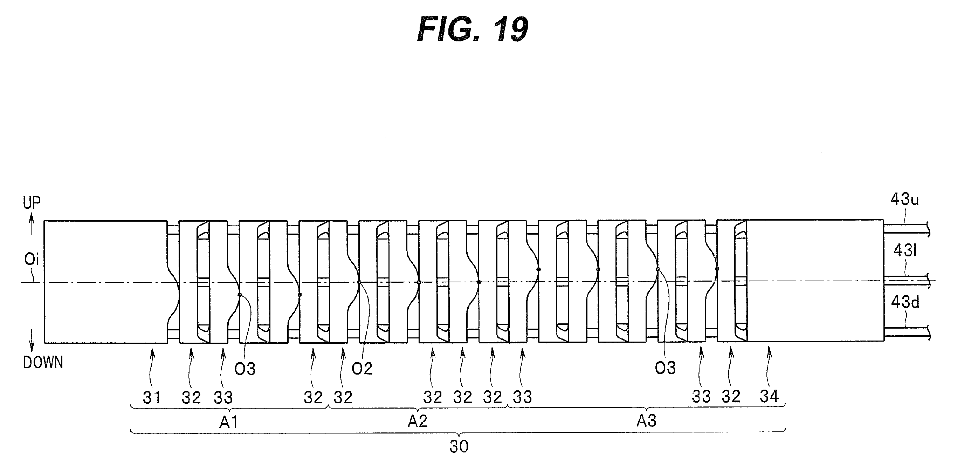

Next, a second modification of the present embodiment will be described with reference to Fig. 19. Note that, in the present modification, for example, first to third bending areas A1 to A3 which show different bending forms are set as bending areas.

That is, in the present modification, for example, third knot rings 33 on which projecting portions 33b are arranged at positions in the left/right direction of the insertion portion 2 which are offset downward, and second knot rings 32 on which projecting portions 32b are arranged in the upward/downward direction of the insertion portion 2 are arranged alternately in the first bending area A1, as shown in Fig. 19.

Further, in a second bending area A2, for example, second knot rings 32 on which projecting portions 32b are arranged in the left/right direction of the insertion portion 2 and second knot rings 32 on which projecting portions 32b are arranged in the upward/downward direction of the insertion portion 2 are arrayed alternately.

Further, in the third bending area A3, for example, third knot rings 33 on which projecting portions 33b are arranged at positions in the left/right direction of the insertion portion 2 which are offset upward, and second knot rings 32 on which projecting portions 32b are arranged in the upward/downward direction of the insertion portion 2 are arranged alternately.

Furthermore, in respective wire inserting portions 32c and 33c of the respective second and third knot rings 32 and 33, corresponding upward/downward pulling wires 43u and 43d or left/right pulling wires 431 and 43r are inserted.

By adopting such a configuration, it is possible, for example, to cause each of bending forms at time of upward bending and at time of downward bending to have further variations.

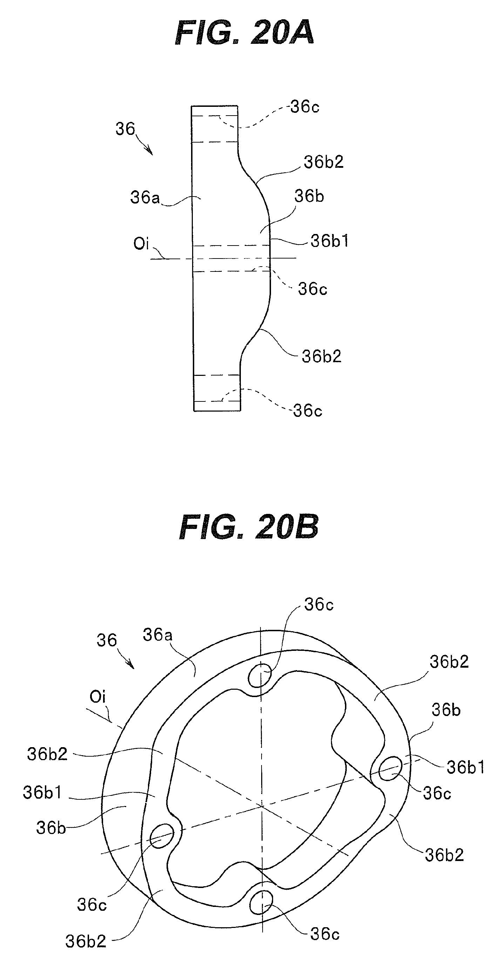

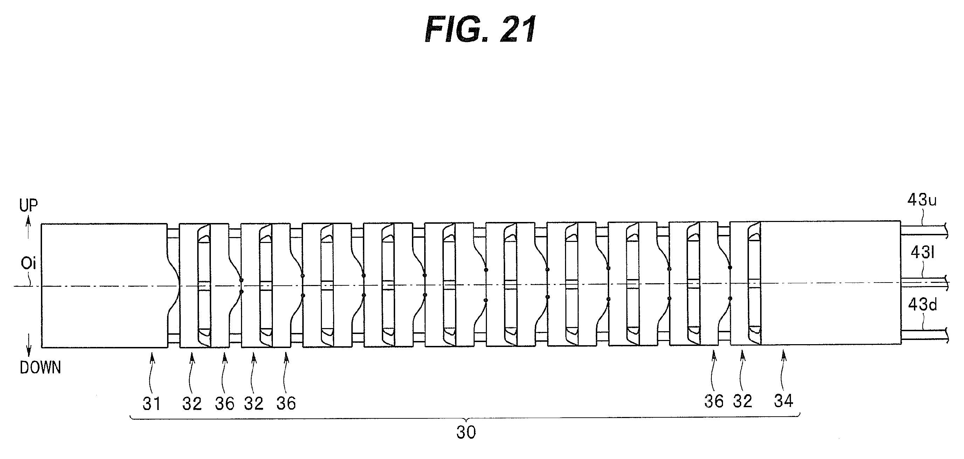

Next, a third modification of the present embodiment will be described with reference to Figs. 20 and 21. Note that, in the present modification, description will be made on a configuration of a sixth knot ring 36 on which contact portions at a time of swinging in one direction and at a time of swinging in the other direction can be set at different positions which are mutually separated.

As shown in Figs. 20A and 20B, the sixth knot ring 36 is configured with a metal member obtained by integrally forming a ring-shaped knot ring body 36a and paired projecting portions 36b projecting from a proximal end side of the knot ring body 36a.

On an inner circumference of the knot ring body 36a, a wire inserting portion 36c is provided, for example, at each rotation position of 45°. That is, on the inner circumference of the knot ring body 36a, two pairs of wire inserting portions 36c, which are axially symmetric to each other relative to a central axis of the knot ring body 36a, are provided at each rotation position of 90°.

The pair of projecting portions 36b constituting the sixth knot ring 36 are configured such that each projecting portion 36b has a flat tip face 36b1 and a pair of projecting curved faces 36b2 continuously connected to both sides of the tip face 36b1, respectively, at its tip portion, for example, as shown in Fig. 20A. Furthermore, centers of the tip faces 36b1 of the respective projecting portions 36b are provided at rotation positions corresponding to respective wire inserting portions 36c of one of the two pairs of wire inserting portions 36c arranged at the axially symmetric positions on the circumference of the knot ring body 36a (see Figs. 20A and 20B).

The sixth knot ring 36 configured as described above is applicable to a knot ring for bending in the upward and downward directions, for example, as shown in Fig. 21. That is, in the knot ring group 30 shown in Fig. 21, for example, second knot rings 32 on which projecting portions 32b are arranged in the left/right direction of the insertion portion 2 and sixth knot rings 36 on which projecting portions 36b are arranged in the upper/lower direction of the insertion portion 2 are arrayed alternately. In this case, a sixth knot ring 36 located nearer to a proximal end side is set to have a wider the tip face 36b in the example shown in Fig. 21.

In the knot ring group 30 configured as described above, for example, when the bending portion 6 bends upward, the projecting curved face 36b2 located above the tip face 36b1 of each projecting portion 36b of the sixth knot ring 36 is caused to come into contact with a knot ring body 32a of an adjoining second knot ring 32, and the sixth knot ring 36 swings with the contact portion as a swing fulcrum. On the contrary, when the bending portion 6 bends downward, the projecting curved face 36b2 located below the tip face 36b1 is caused to come into contact with a knot ring body 32a of an adjoining second knot ring 32, and the sixth knot ring 36 swings with the contact portion as a swing fulcrum.

By adopting such a configuration, an amount of offset of a swing fulcrum relative to the central axis Oi of the insertion portion 2 can be equally set for both of one and the other swing direction of the sixth knot ring 36.

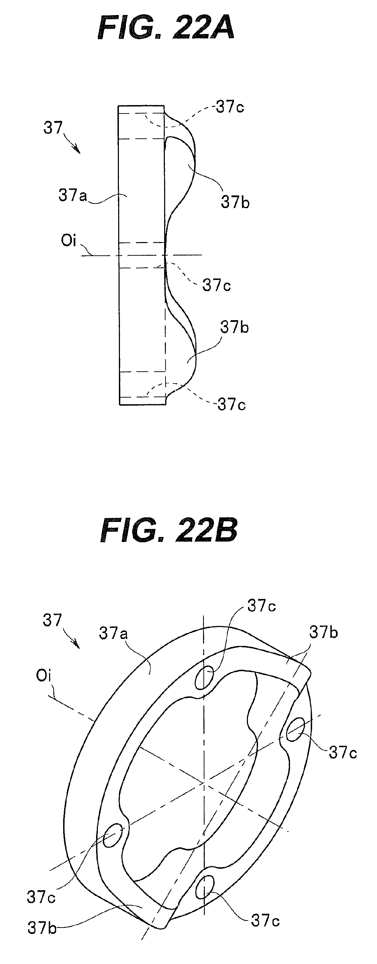

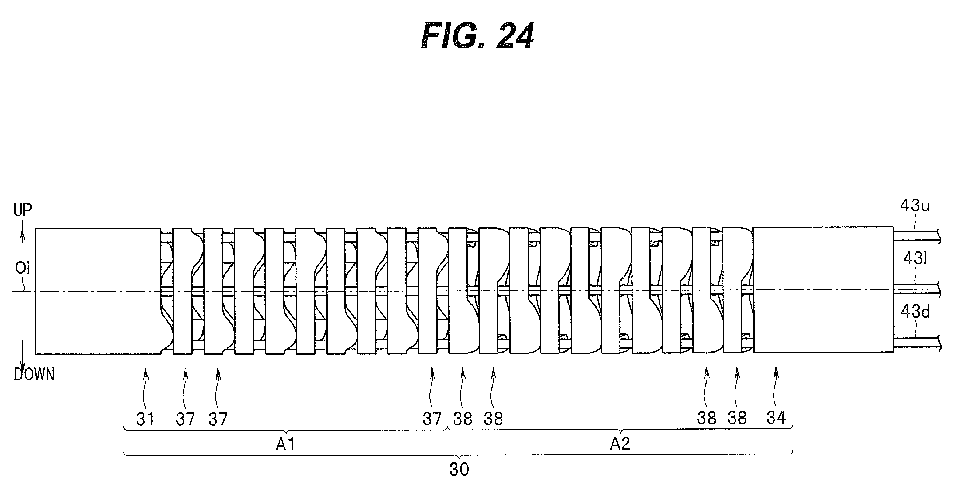

Next, a fourth modification of the present embodiment will be described with reference to Figs 22 to 24. Note that, in the present modification, description will be made on an example in which the knot ring group 30 is configured with use of seventh and eighth knot rings 37, 38 with a wire inserting portion and projecting portions arranged at different rotation positions on a circumference of a knot ring body.

As shown in Figs. 22A and 22B, the seventh knot ring 37 is configured with a metal member obtained by integrally forming a ring-shaped knot ring body 37a and paired projecting portions 37b projecting from a proximal end side of the knot ring body 37a.

On an inner circumference of the knot ring body 37a, a wire inserting portion 37c is provided, for example, at each rotation position of 45°. That is, on the inner circumference of the knot ring body 37a, two pairs of wire inserting portions 37c, which are axially symmetric to each other relative to a central axis of the knot ring body 37a, are provided at each rotation position of 90°.

The pair of projecting portions 37b constituting the seventh knot ring 37 are configured with tongue-piece-shaped members having arc-shaped or parabolic-shaped projecting curved faces at their tip portions and projecting to a proximal end side. These projecting portions 37b are provided facing each other, for example, so as to be axially symmetric to each other relative to the central axis Oi of the insertion portion 2 (that is the central axis of the knot ring body 37a). Furthermore, the respective projecting portions 37b are provided at rotation positions which do not correspond to any wire inserting portion 37c on the circumference of the knot ring body 37a. More specifically, each projecting portion 37b is arranged at an intermediate position between two adjoining wire inserting portions 37c on the circumference of the knot ring body 37a.

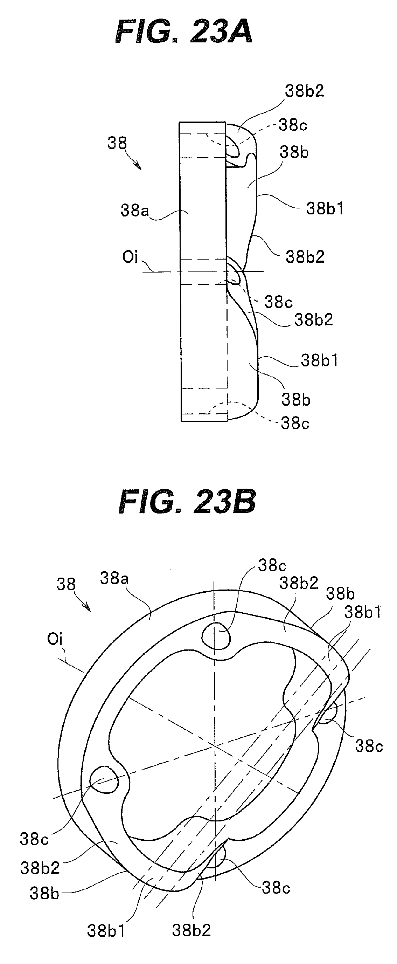

Further, as shown in Figs. 23A and 23B, the eighth knot ring 38 is configured with a metal member obtained by integrally forming a ring-shaped knot ring body 38a and paired projecting portions 38b projecting from a proximal end side of the knot ring body 38a.

On an inner circumference of the knot ring body 38a, a wire inserting portion 38c is provided, for example, at each rotation position of 45°. That is, on the inner circumference of the knot ring body 38a, two pairs of wire inserting portions 38c, which are axially symmetric to each other relative to a central axis of the knot ring body 38a, are provided at each rotation position of 90°.

The pair of projecting portions 38b constituting the eighth knot ring 38 are configured such that each projecting portion 38b has a flat tip face 38b1 and a pair of projecting curved faces 38b2 continuously connected to both sides of the tip face 38b1, respectively, at its tip portion. Furthermore, centers of the tip faces 38b1 of the respective projecting portion 38b are provided at rotation positions which do not correspond to any wire inserting portion 38c on the circumference of the knot ring body 38a. More specifically, each projecting portion 38b is arranged so that the center of the tip face 38b1 is located at an intermediate position between two adjoining wire inserting portions 38c on the circumference of the knot ring body 38a.

Between the seventh and eighth knot rings 37, 38 configured as described above, the seventh knot rings 37 are arrayed in a first bending area A1 set on a distal end side of the knot ring group 30, and the eighth knot rings 38 are arrayed in a second bending area A2 set on the proximal end side of the knot ring group 30. More specifically, in the first bending area A1 of the knot ring group 30, the respective seventh knot rings 37 are arrayed so that respective projecting portions 37b are alternately positioned between adjoining seventh knot rings 37. Further, the eighth knot rings 38 are arrayed so that respective projecting portions 38b are alternately positioned between adjoining eighth knot rings 38.

In such a configuration, when any of the upward/downward pulling wires 43u and 43d or the left/right pulling wires 431 and 43r is pulled, the respective knot rings 37, 38 swing in inclined directions relative to pulling directions alternately. However, by inclination components being offset between adjoining knot rings, bending in a predetermined bending direction is caused. In this case, since swing fulcrums set for projecting portions 38b of the eighth knot rings 38 are offset from the central axis of the insertion portion 2, the first bending area A1 is preferentially bent prior to the second bending area A2.

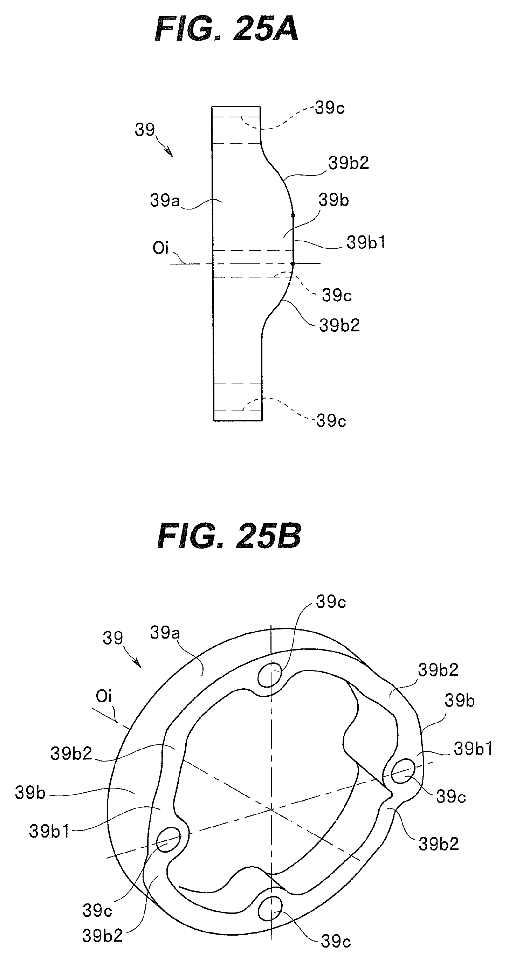

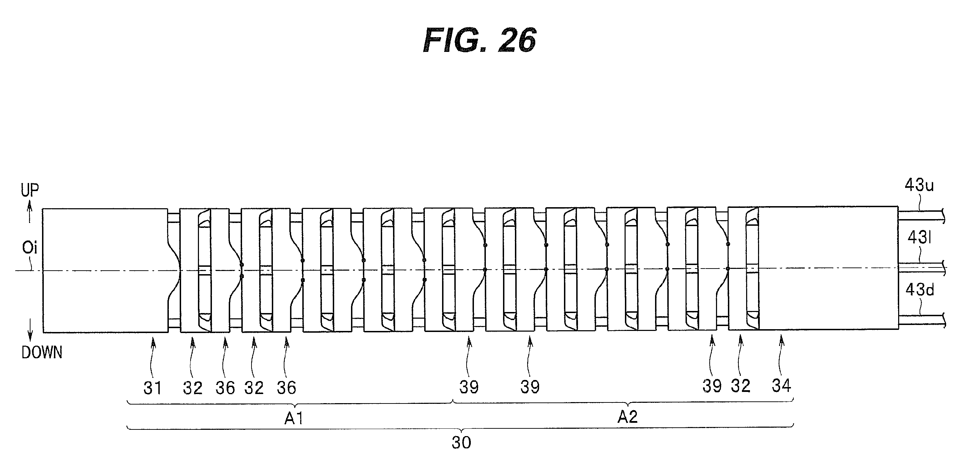

Next, a fifth modification of the present embodiment will be described with reference to Figs. 25 and 26. Note that, in the present modification, description will be made on a configuration of a ninth knot ring 39 on which contact portions at a time of swinging in one direction and at a time of swinging in the other direction can be set at different positions which are mutually separated.

As shown in Figs. 25A and 25B, the ninth knot ring 39 is configured with a metal member obtained by integrally forming a ring-shaped knot ring body 39a and paired projecting portions 39b projecting from a proximal end side of the knot ring body 39a.

On an inner circumference of the knot ring body 39a, a wire inserting portion 39c is provided, for example, at each rotation position of 45°. That is, on the inner circumference of the knot ring body 39a, two pairs of wire inserting portions 39c, which are axially symmetric to each other relative to a central axis of the knot ring body 39a, are provided at each rotation position of 90°.

The pair of projecting portions 39b constituting the ninth knot ring 39 are configured such that each projecting portion 39b has a flat tip face 39b1 and a pair of projecting curved faces 39b2 continuously connected to both sides of the tip face 39b1, respectively, at its tip portion, for example, as shown in Fig. 25A. Furthermore, the respective projecting portions 39b are set at such positions that an axial line connecting centers of the respective tip faces 39b1 is offset relative to the central axis Oi of the insertion portion 2. In this case, an amount of offset of each projecting portion 39b is set so that one pair of wire inserting portions 39c of the two pairs of wire inserting portions 39c arranged at the axially symmetric positions are arranged within a rotation position corresponding to between starting points of the pair of projecting curved faces 39b2 continuously connected to each tip face 39b1 on the circumference of the knot ring body 39a. In the present modification, more specifically, the amount of offset for each projecting portion 39b is set so that starting points of projecting curved faces 39b2 located on opposite sides, among projecting curved faces 39b2 of a pair of projecting curved faces 39b2 continuously connected to both sides of each tip face 39b1, are located at rotation positions corresponding to one of the two pairs of wire inserting portions 39c arranged at the axially symmetric positions, on the circumference of knot ring body 39a (see Figs. 25A and 25B).

The ninth knot ring 39 configured as described above is applicable to a knot ring for bending in the upward and downward directions in a second bending area A2, for example, as shown in Fig. 26. In the shown example, the respective ninth knot rings 39 and second knot rings 32 used as knot rings for bending in the right and left directions are arranged alternately. Furthermore, each ninth knot ring 39 is arranged so that the projecting portions 39b are offset upward relative to the central axis Oi and so that the starting points of the projecting curved faces 39b2 continuously connected to opposite sides of each tip face 39b1 are located at rotation positions corresponding to insertion positions of the left/right pulling wires 431 and 43r on the circumference of the knot ring body 39a. Note that the configuration of a first area A1 is substantially similar to the configuration shown in the third modification described above.

According to such a configuration, it is possible to cause the curvature radius of the bending portion 6 in the upward/downward direction in the second bending portion A2 to be different between an upward position and a downward position at the bending to the upward and downward directions. On the other hand, since the respective projecting portions 39b of the ninth knot rings 39 are arranged so that the respective tip faces 39b1 are arranged at rotation positions corresponding to the left/right pulling wires 431 and 43r, it is possible to, when the bending portion 6 is in a state of not bending, cause at least a part of the respective projecting portions 39b of the ninth knot rings 39 to be in contact with an adjoining second knot ring 32 at positions near the left/right pulling wires 431 and 43r and axially symmetric to each other relative to the central axis Oi of the insertion portion 2. Therefore, even if the projecting portions 39b are offset from the axially symmetric positions relative to the central axis Oi, it is possible to favorably keep load balance, and it is possible to stably hold a straight state of the second bending area.

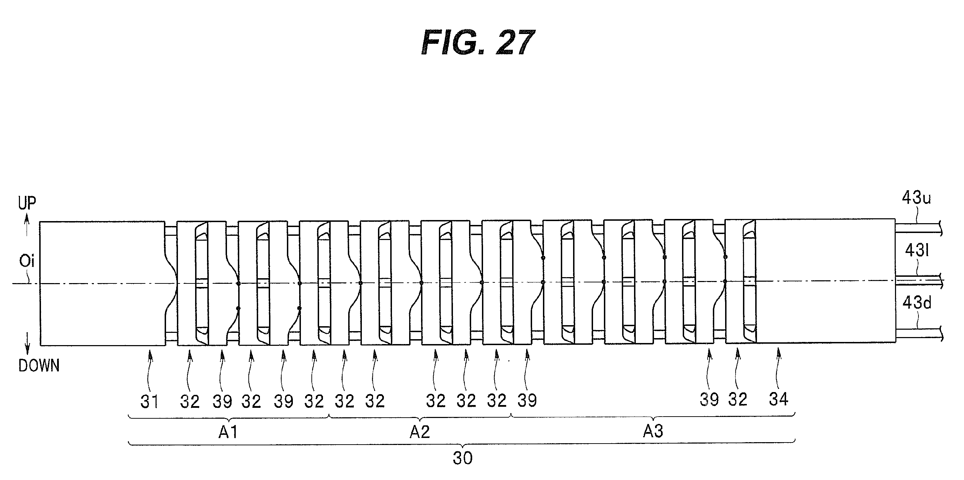

Next, a sixth modification of the present embodiment will be described with reference to Fig. 27. Note that, in the present modification, for example, first to third bending areas A1 to A3 which show different bending forms are set as bending areas.

That is, in the present modification, for example, ninth knot rings 39 on which projecting portions 39b are arranged at positions in the right and left direction of the insertion portion 2 which are offset downward, and second knot rings 32 on which the projecting portions 32b are arranged in the upward/downward direction of the insertion portion 2 are arranged alternately in the first bending area A1, as shown in Fig. 27.

Further, in a second bending area A2, for example, second knot rings 32 on which projecting portions 32b are arranged in the left/right direction of the insertion portion 2 and second knot rings 32 on which projecting portions 32b are arranged in the upward/downward direction of the insertion portion 2 are arrayed alternately.

Further, in the third bending area A3, for example, ninth knot rings 39 on which projecting portions 39b are arranged at positions in the left/right direction of the insertion portion 2 which are offset upward, and second knot rings 32 on which projecting portions 32b are arranged in the upward/downward direction of the insertion portion 2 are arranged alternately.

Furthermore, in the respective wire inserting portions 32c and 39c of the respective second and ninth knot rings 32 and 39, corresponding upward/downward pulling wires 43u and 43d or left/right pulling wires 431 and 43r are inserted.

By adopting such a configuration, it is possible, for example, to cause each of bending forms at time of upward bending and at time of downward bending to have further variations.

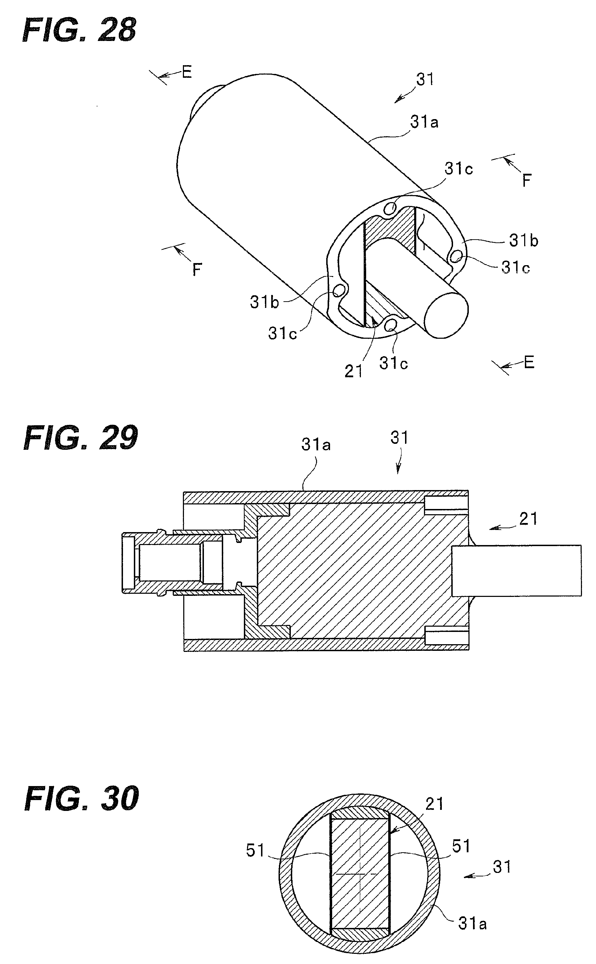

Next, a seventh modification of the present embodiment will be described with reference to Figs 28 to 30. Note that, in the present modification, description will be made on an example of a configuration in which the knot ring body 31a of a first knot ring 31 is caused to play a role of a reinforcing frame of the image pickup unit 21.

As shown in Figs. 28 and 30, a pair of partitions 51 facing each other are provided inside the knot ring body 31a, and an inner area surrounded by the partitions 51 is formed as a housing chamber of the image pickup unit 21 (see Figs. 28 and 29). Further, an outer area of each partition 51 is formed as a housing chamber of the light guide fiber bundle 22. For example, the wires 22a exposed from the exterior tube 22b can be immediately inserted into the housing chamber (not shown).

By adopting such a configuration, a dedicated reinforcing frame is unnecessary, and an outer diameter of the distal end rigid portion 5 can be reduced more.

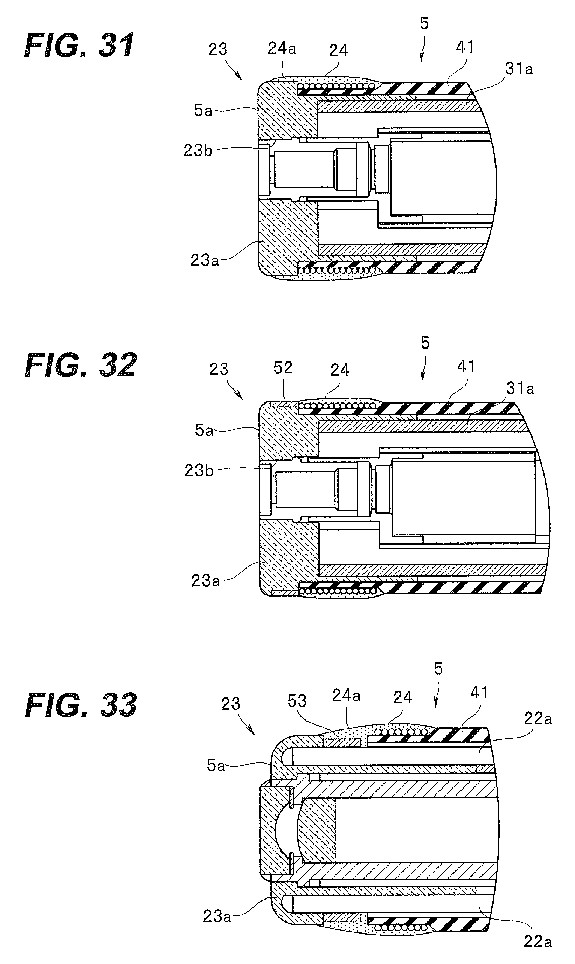

Next, an eighth modification of the present embodiment will be described with reference to Fig. 31. Note that, in the present modification, description will be made on an example of improving durability of the distal end portion body 23 configured with transparent resin material or the like more.

As shown in Fig. 31, the first bobbin portion 24 for bonding and fixing the distal end portion of the bending rubber 41 to the distal end portion body 23 is provided near to the distal end of the distal end portion body 23. As an adhesive 24a used for the first bobbin portion 24, for example, an epoxy adhesive with excellent chemical resistance against cleaning solution, disinfecting liquid and the like is adopted, and the adhesive 24a extends to the distal end side so as to cover an outer circumferential face of the distal end portion body 23. Thereby, almost all areas of the distal end portion body 23 excluding an end face of the distal end portion body 23 (that is, the observation portion 5a of the distal end rigid portion 5) are covered with the bending rubber 41 or the adhesive 24a, and durability at time of cleaning or the like is improved.

Next, a ninth modification of the present embodiment will be described with reference to Fig. 32. Note that, in the present modification, description will be made on an example of improving the durability of the distal end portion body 23 configured with transparent resin material or the like more.

As shown in Fig. 32, the first bobbin portion 24 for bonding and fixing the distal end portion of the bending rubber 41 to the distal end portion body 23 is provided near to the distal end of the distal end portion body 23. On a distal end side of the first bobbin portion 24, a thin metal pipe 52 is fit to an outer circumference of the distal end portion body 23. The metal pipe 52 is bonded and fixed to the distal end portion body 23. Thereby, almost all areas of the distal end portion body 23 excluding an end face of the distal end portion body 23 (that is, the observation portion 5a of the distal end rigid portion 5) are covered with the bending rubber 41 or the metal pipe 52, and the durability at time of cleaning or the like is improved.

Next, a tenth modification of the present embodiment will be described with reference to Fig. 33. Note that, in the present modification, description will be made on an adhesion structure of the light guide fiber bundle 22 whose wires 22a are inserted in the light guide holding holes 23c of the distal end portion body 23 configured with transparent resin material or the like.

As shown in Fig. 33, a ring-shaped spacer 52 whose inner circumference side the wires 22a of the light guide fiber bundle 22 are to be in contact with is interposed between a proximal end portion of the distal end wall portion 23a of the distal end portion body 23 and the distal end portion of the bending rubber 41. A distal end side of the spacer 53 is bonded and fixed to the distal end wall portion 23a via the adhesive 24a used for the first bobbin portion 24. Further, a slight gap is provided between a proximal end side of the spacer 53 and a distal end side of the bending rubber 41, and the wires of the light guide fiber bundle 22 are bonded and fixed to the spacer 53 via the adhesive 24a caused to flow in through the gap.

By such a configuration, the wires 22a of the light guide fiber bundle 22 are fixed to the distal end portion body 23 via the spacer 53, and a bonded portion of the wires 22a is set at a position away from the light guide holding holes 23c. Therefore, for example, even in a case where the distal end wall portion 23a is formed relatively thin, and the light guide holding holes 23c are formed as shallow holes, it is possible to prevent the adhesive for bonding and fixing the wires 22a to the distal end portion body 23 from flowing to a distal end side of the light guide holding holes 23c.

The present invention is not limited to each embodiment described above, and various modifications and changes are possible. The various variations and changes are also within the technical scope of the present invention. For example, though a configuration for transforming a bending form mainly in upward and downward directions has been shown in the embodiment and respective modifications described above, the present invention is not limited to the configuration. For example, a similar configuration is, of course, applicable to right and left directions. Further, though an example of applying the present invention to a bending portion bendable in four axial directions of upward and downward directions and right and left directions has been described in the embodiment and respective modifications described above, the present invention is not limited to that. For example, the present invention is applicable to a bending portion bendable in two axial directions. Further, the configurations of the embodiment and respective modifications described above may be, of course, appropriately combined.

The present application is filed, claiming priority based on Patent Application No. 2013-248475 filed to Japan on November 29, 2013, the content of which is incorporated in the specification, claims, drawings of the present application by reference thereto.

## Claims

1. A bending portion of an endoscope comprising a knot ring group in which a plurality of knot rings are arrayed in line along a central axis of an insertion portion and a pulling wire capable of pulling the knot ring located on a distal end side of the knot ring group from a proximal end side, the knot ring group being caused to perform a bending motion by pulling force of the pulling wire, wherein
each of the knot rings has a ring-shaped knot ring body and paired projecting portions projecting from one end face of the knot ring body and swingably contacting the other end face of another adjoining knot ring body; and
as for at least one of the respective knot rings, an axial line connecting swing fulcrums formed at respective portions of contact with the other end face of the other knot ring body adjoining the pair of projecting portions is offset relative to a central axis of the insertion portion by an amount of offset different from an amount of offset of an axial line connecting the swing fulcrums of any other of the knot rings.

2. The bending portion of an endoscope according to claim 1, wherein, on the at least one of the respective knot rings, the pair of projecting portions have a projecting curved face at a tip portion and are offset from axially symmetric positions relative to the central axis of the insertion portion.

3. The bending portion of an endoscope according to claim 2, wherein, on the at least one of the respective knot rings, a height of the one end face of the knot ring body continuously connected to the pair of projecting portions is set to be relatively higher on a side where the pair of projecting portions are offset than on a side where the pair of projecting portions are not offset.

4. The bending portion of an endoscope according to claim 1, wherein, on the at least one of the respective knot rings, the pair of projecting portions have a flat tip face and a pair of projecting curved faces continuously connected to both sides of the tip face at a tip portion;
on each of the projecting portions, one of the pair of projecting curved faces constitutes the contact portion at a time of the knot ring swinging in one direction; and
on each of the projecting portions, the other of the pair of projecting curved faces constitutes the contact portion at a time of the knot ring swinging in the other direction.

5. The bending portion of an endoscope according to claim 4, wherein
the knot ring body has a wire inserting portion through which the pulling wire is inserted; and
the wire inserting portion is arranged within a rotation position corresponding to between starting points of the pair of projecting curved faces continuously connected to the tip face on a circumference of the knot ring body.

6. The bending portion of an endoscope according to claim 1, wherein
the knot ring body has a wire inserting portion through which the pulling wire is inserted; and
the wire inserting portion is arranged at a rotation position different from the projecting portions on a circumference of the knot ring body.
